# EUROPÄISCHE PATENTANMELDUNG

(11) **EP 1 772 106 A1**
(43) Veröffentlichungstag der Anmeldung: **11.04.2007**
(21) Anmeldenummer: 05021794.2
(22) Anmeldetag: 06.10.2005
(51) Int. Cl.: A61B 17/17

(54) **Instrument zum Vorbereiten und/oder Bearbeiten eines Femurkopfes**

(71) Anmelder: Zimmer GmbH, 8404 Winterthur (CH)
(72) Erfinder: Willi, Roland, 8413 Neftenbach (CH); Hinder, Marcel, 9000 St. Gallen (CH); Marchione, Andreas, 8404 Winterthur (CH)
(74) Vertreter: Manitz, Finsterwald & Partner GbR

(57) **Zusammenfassung**

Instrument zum Vorbereiten und/oder Bearbeiten eines Femurkopfes, insbesondere zum Festlegen einer bezüglich des Femurhalses zentralen Bohrung für ein bevorzugt draht- oder stabförmiges Führungselement eines Bearbeitungswerkzeuges, mit zwei nach Art einer Schere oder Zange gelenkig miteinander verbundenen Tasthebeln (13,15), die jeweils auf der einen Seite des Gelenkbereiches (17) eine Tastbacke (23,25) zum Ertasten des Femurhalses (19) und auf der anderen Seite des Gelenkbereiches einen Betätigungsabschnitt (33,35) zum Öffnen und Schließen der Tastbacken aufweisen, und mit einer eine Peil und/oder Bohrachse (21) definierenden Funktionseinheit (27), die mit den Tasthebeln (13,15) derart verbunden ist, dass die Peil- und/oder Bohrachse (21) unabhängig von der Stellung der Tasthebel (13,15) stets in einer durch den Gelenkbereich (17) und mittig zwischen den Tastbacken (23,25) hindurch verlaufenden Mittelebene (12) liegt.

## Beschreibung

Die Erfindung betrifft ein Instrument zum Vorbereiten und/oder Bearbeiten eines Femurkopfes.

Insbesondere im Rahmen der vorbereitenden Bearbeitung des Femurkopfes beim Einsetzen von Hüftprothesen müssen die Bearbeitungsinstrumente auf definierte Weise positioniert und geführt werden, und zwar in Abhängigkeit von der konkreten Form des Femurkopfes. Von besonderer Bedeutung ist eine exakt auf den jeweiligen Patienten abgestimmte Femurkopfbearbeitung im Fall eines so genannten "re-surfacing", bei dem nicht der gesamte natürliche Femurkopf ersetzt, sondern lediglich eine äußere Schicht an Knochenmaterial entfernt wird, um anschließend ein kappenförmiges Femurimplantat auf den verbliebenen Femurstumpf aufzusetzen, die mit der Hüftschale der Prothese zusammenwirkt. Gerade bei derartigen Femurkappen muss sichergestellt werden, dass die auftretenden Belastungen vom Femurhals aufgenommen werden, um unter anderem einen sicheren Halt der Femurkappe auf dem bearbeiteten Femurkopf zu gewährleisten. Hierzu sollte die Ausrichtung der Bearbeitungsinstrumente in Bezug auf den Femurhals erfolgen.

Eine sich am Femurhals orientierende, genaue Bestimmung einer optimalen Ausrichtung der Bearbeitungsinstrumente wird in der Praxis dadurch erschwert, dass in einigen Fällen der Femurkopf nicht gerade bzw. symmetrisch auf dem Femurhals sitzt. Gerade bei älteren Patienten kommt es vor, dass sich der Femurkopf im Laufe der Zeit durch die Belastung aus der Halsachse heraus "verschoben" bzw. geneigt hat. In diesen Fällen kann es zu einer unvorteilhaften Fehlausrichtung der Femurkappe kommen, wenn sich der Operateur bei der Ausrichtung der Bearbeitungsinstrumente am Femurkopf - und nicht am Femurhals - orientiert, da dann die auf die Femurkappe einwirkenden Belastungen nicht optimal vom Femurhals aufgenommen werden können. Dem Femurhals als Bezug für die Ausrichtung der Bearbeitungsinstrumente kommt also im Vorfeld der Femurkopfbearbeitung eine große Bedeutung zu.

Instrumente zum Ertasten des Femurhalses und Festlegen der Orientierung der anschließend zu verwendenden Bearbeitungsinstrumente sind grundsätzlich bekannt, erweisen sich hinsichtlich einer einfachen Handhabung und eines geringen Platzbedarfes in manchen Fällen aber zum Teil noch als unbefriedigend.

Aufgabe der Erfindung ist es daher, ein Instrument der eingangs genannten Art zu schaffen, mit dessen Hilfe auf einfache und zuverlässige Weise anhand des Femurhalses eine für die nachfolgende Anbringung eines insbesondere kappenartigen Femurkopfimplantates optimale Ausrichtung der für die erforderliche Vorbereitung bzw. Bearbeitung des Femurkopfes verwendeten Instrumente festgelegt werden kann.

Die Lösung dieser Erfindung erfolgt durch die Merkmale des Anspruchs 1.

Erfindungsgemäß umfasst das Instrument zwei nach Art einer Schere oder Zange gelenkig miteinander verbundene Tasthebel oder Klemmen, die jeweils auf der einen Seite des Gelenkbereiches eine Tastbacke zum Ertasten des Femurhalses und auf der anderen Seite des Gelenkbereiches einen Betätigungsabschnitt zum Öffnen und Schließen der Tastbacken aufweisen, wobei außerdem eine eine Peil- und/oder Bohrachse definierende Funktionseinheit vorgesehen ist, die mit den Tasthebeln derart verbunden ist, dass die Peil- und/oder Bohrachse unabhängig von der Stellung der Tasthebel stets in einer durch den Gelenkbereich und mittig zwischen den Tastbacken hindurch verlaufenden Mittelebene liegt.

Die scheren- oder zangenartige Ausgestaltung des erfindungsgemäßen Instrumentes bietet dem Operateur einen Zugang "von oben", d.h. zumindest näherungsweise in Richtung der Femurhalsachse, indem die Tastbacken so weit geöffnet werden, dass sie an dem Femurkopf vorbei gelangen können. Ein Arbeiten "von oben" ist äußerst einfach und benötigt vor allem wenig Platz. Das anschließende Ertasten des Femurhalses erfolgt einfach durch Schließen der Tastbacken. Dabei kann der Operateur den Übergang zwischen Femurkopf und Femurhals bzw. den Halsansatz zuverlässig ertasten, indem er z.B. mit den Tastbacken gewissermaßen die Kontur des Femur im Kopfbereich "abfährt". Hierzu sind keine raumgreifenden Bewegungen des Instrumentes erforderlich, sondern das Instrument braucht hierbei lediglich relativ geringfügig geöffnet und geschlossen zu werden.

Wenn die Tastbacken am Femurhals oder am Halsansatz anliegen, ist erfindungsgemäß automatisch sichergestellt, dass auch die Femurhalsachse sich zumindest näherungsweise in der Mittelebene des Instrumentes erstreckt, die durch den Gelenkbereich der Tasthebel bzw. deren Drehachse und mittig zwischen den Tastbacken hindurch verläuft. Damit ist ein klar definierter Bezug für die Funktionseinheit des erfindungsgemäßen Instrumentes geschaffen, so dass mit der Funktionseinheit dann auf definierte Art und Weise Peilvorgänge zur Feinausrichtung durchgeführt werden können bzw. eine Bohrung insbesondere für ein draht- oder stiftförmiges Führungselement ausgebildet werden kann, an welchem die späteren Bearbeitungswerkzeuge relativ zum Femurkopf geführt werden.

Die Vorgehensweise bei der Ausrichtung des erfindungsgemäßen Instrumentes am Femur ist als ein in den meisten Fällen iterativer Vorgang zu verstehen, bei dem nach einem ersten Ansetzen der Tastbacken ggf. noch Korrekturen der Lage des Instrumentes relativ zum Femurkopf erforderlich sind. Hierzu kann die Funktionseinheit dienen, indem sie mit Peileinrichtungen versehen bzw. gekoppelt wird. Durch die erfindungsgemäße Ausgestaltung des Instrumentes ist dabei in jeder Stellung mit am Femurhals bzw. am Halsansatz anliegenden Tastbacken zumindest bezüglich der Mittelebene stets eine optimale Ausrichtung in Bezug auf die Femurhalsachse gewährleistet, wodurch die Ausrichtung der anschließend zu verwendenden Bearbeitungsinstrumente für den Operateur erheblich vereinfacht wird.

Weitere Ausführungsbeispiele der Erfindung sind auch in den abhängigen Ansprüchen, der Beschreibung sowie der Zeichnung angegeben.

Vorzugsweise umfasst die Funktionseinheit eine Bohrlehre, dessen Bohrachse parallel zu einer von den beiden Tasthebeln aufgespannten Hebelebene verläuft und in der Mittelebene liegt. Auf diese Weise kann nach erfolgter Ausrichtung des Instrumentes am Femur eine zentrale Bohrung im Femurkopf ausgebildet werden, in die dann ein draht- oder stabförmiges Führungselement eingebracht werden kann, um mit dessen Hilfe Bearbeitungswerkzeuge, insbesondere Fräser, auf zuverlässige Weise in einer hinsichtlich der Lage der Femurkappe relativ zum Femurkopf optimalen Orientierung heranzuführen und während der Bearbeitung zu positionieren und zu bewegen.

In einem weiteren bevorzugten Ausführungsbeispiel sind die Tasthebel um eine feste Drehachse gegeneinander verdrehbar und mit Abstand von der Drehachse zusätzlich über wenigstens eine Gelenkhebelanordnung miteinander verbunden. Bevorzugt umfasst die Gelenkhebelanordnung eine in der Mittelebene liegende Linearführung für sich beim Öffnen und Schließen der Tastbacken bewegende Gelenkhebel.

Hierbei kann ein Halter vorgesehen sein, der die Funktionseinheit trägt und mit den Tasthebeln verbindet und an dem die Linearführung ausgebildet ist. Die Linearführung ist vorzugsweise in Form eines Langlochs vorgesehen.

In einer Weiterbildung können die Tastbacken an verschwenkbaren Endabschnitten der Tasthebel ausgebildet sein. Dabei können die verschwenkbaren Endabschnitte durch eine Gelenkhebelanordnung miteinander verbunden sein. Diese Gelenkhebelanordnung kann zusätzlich zu einer oberhalb des Gelenkbereiches gelegenen Gelenkhebelanordnung, die insbesondere eine Linearführung aufweist, vorgesehen sein.

Diese die verschwenkbaren Endabschnitte miteinander verbindende Gelenkhebelanordnung ist in einer bevorzugten Weiterbildung als ein Parallelogrammgelenk ausgeführt. Hierdurch kann erreicht werden, dass beim Öffnen und Schließen des erfindungsgemäßen Instrumentes unabhängig von der Öffnungsweite die verschwenkbaren Endabschnitte der Tasthebel - bzw. diejenigen Teile der verschwenkbaren Endabschnitte, die parallel zu einer von den Tasthebeln aufgespannten Ebene liegen - stets parallel zueinander verlaufen. Hierzu ist bei optimierter Handhabung des erfindungsgemäßen Instrumentes dessen Platzbedarf minimiert.

Die Funktionseinheit kann mit wenigstens einem Peilausleger versehen sein, mit dem zumindest ein Peilstab derart gekoppelt werden kann, dass sich der Peilstab parallel zur Peil- und/oder Bohrachse der Funktionseinheit erstreckt. Dabei kann ein Peilausleger verschwenkbar ausgeführt sein, und zwar um eine Achse, die parallel versetzt zur Peil- und/oder Bohrachse der Funktionseinheit verläuft oder mit dieser Achse zusammenfällt.

Des Weiteren kann das erfindungsgemäße Instrument einen Tastarm zum Abtasten des Femurhalses und/oder des Übergangs zwischen Femurkopf und Femurhals umfassen, wobei dieser Tastarm um die Peil- und/oder Bohrachse der Funktionseinheit verschwenkbar ist. Mit einem solchen Tastarm kann ein so genanntes "Auskreiseln" durchgeführt werden, wodurch der Operateur die Ausrichtung des Instrumentes relativ zu dem Femurkopf unter Berücksichtigung zumindest eines wesentlichen Teils des gesamten Umfangs kontrollieren kann.

Des Weiteren können an den Tastbacken Peilstifte angebracht sein, deren Verbindungslinie senkrecht zur Mittelebene steht und die Bohrachse kreuzt. Diese Peilstifte ermöglichen eine Ausmittung im Halsbereich des Femurkopfes, wenn das Instrument angesetzt wird.

Des Weiteren kann das erfindungsgemäße Instrument mit einem verstellbaren Hilfsanschlag versehen sein, mit dem ein zusätzlicher Bezugspunkt für den Operateur während des Ausrichtvorgangs geschaffen werden kann. Dieser Hilfsanschlag kann in der Mittelebene vorzugsweise in zwei senkrecht zueinander verlaufenden Richtungen verstellbar sein.

Die Erfindung wird im Folgenden beispielhaft unter Bezugnahme auf die Zeichnung beschrieben. Es zeigen:
- Fig. 1 - 4: verschiedene Ansichten eines Ausführungsbeispiels eines erfindungsgemäßen Instrumentes,

- Fig. 5: das Instrument der Fig. 1 - 4 an einem zu bearbeitenden Femurkopf, und
- Fig. 6 und 7: verschiedene Ansichten einer weiteren Ausführungsform eines erfindungsgemäßen Instrumentes.

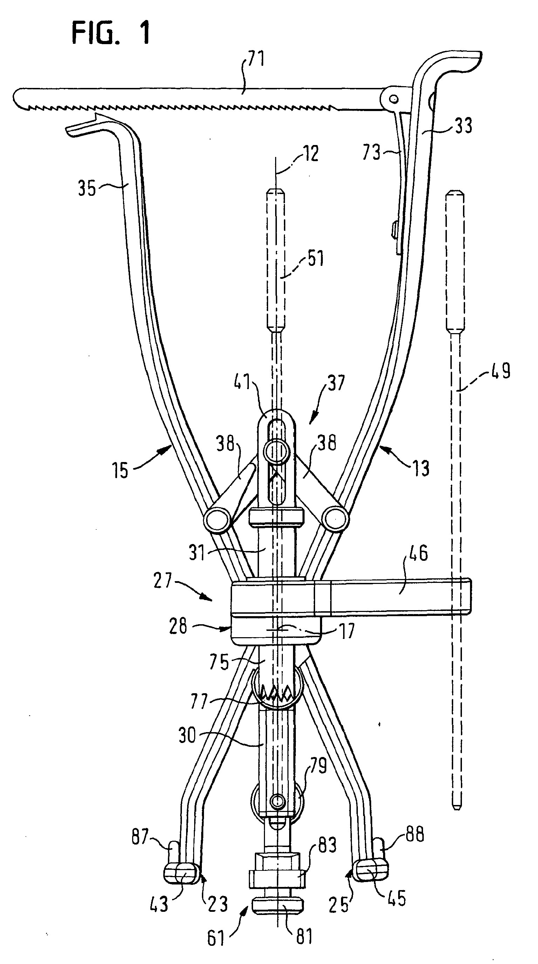

Das in den Fig. 1 - 5 dargestellte Ausführungsbeispiel eines erfindungsgemäßen Instrumentes umfasst zwei Tasthebel oder Klemmen 13, 15, die wie bei einer Schere oder Zange gelenkig miteinander verbunden sind. Das Instrument ist insofern von X-förmiger Gestalt, wobei der eine Drehachse 17 definierende Gelenkbereich nicht in der Mitte der beiden Tasthebel 13, 15 liegt, sondern diese jeweils etwa im Verhältnis von 1:2 bis 1:3 teilt.

Einer der beiden längeren oberen Betätigungsabschnitte 33, 35 ist mit einem Rastarm 71 versehen, der an seiner zur Drehachse 17 weisenden Seite mit einer Rastverzahnung versehen ist, über die der Rastarm 71 mit dem freien Ende des anderen Betätigungsabschnitts 35 zusammenwirkt, um während einer Schließbewegung des Instrumentes die jeweils erreichte Stellung durch Verrastung zu halten. Die hierfür notwendige Vorspannung des Rastarmes 71 wird mittels einer am Betätigungsabschnitt 33 befestigten Feder 73 erreicht.

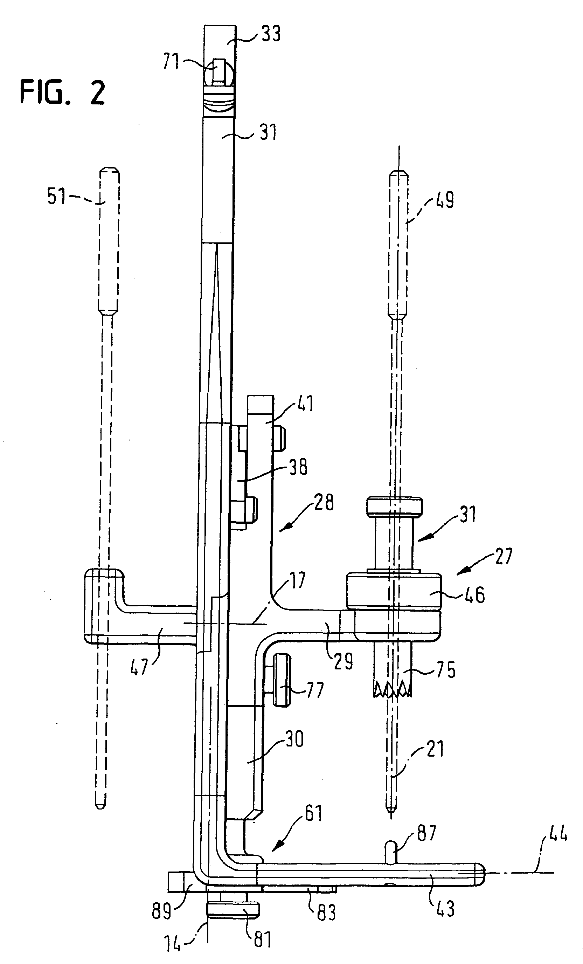

Die Tasthebel 13, 15 spannen eine Hebelebene 14 auf (Fig. 2), senkrecht zu welcher sich Tastabschnitte 43, 45 erstrecken, die an ihren einander zugewandten Innenseiten als Tastbacken 23, 25 ausgebildet sind.

Ein Halter 28 auf der einen Seite der Hebelebene 14 und ein hinterer Peilausleger 47 auf der anderen Seite der Hebelebene 14 sind mittels einer durch Bohrungen in den Tasthebeln 13, 15 im Gelenkbereich 17 hindurch verlaufende Steckverbindung miteinander verbunden. Die Steckachse fällt mit der Drehachse 17 zusammen. Der hintere Peilausleger 47 weist ein abgewinkeltes Endstück auf, durch das ein in Fig. 1 und 2 gestrichelt angedeuteter Peilstab 51 gesteckt werden kann, der sich parallel zur Hebelebene 14 erstreckt und in einer nachstehend näher erläuterten Mittelebene 12 (Fig. 1) liegt.

Der Halter 28 weist eine T-förmige Grundform auf, wobei der senkrechte Fuß des "T" von einem sich längs der Drehachse 17 erstreckenden Träger 29 für eine nachstehend näher erläuterte Funktionseinheit 27 gebildet wird. Der obere, auf der Betätigungsseite der Drehachse 17 gelegene Teil des Querbalkens des "T" ist mit der bereits erwähnten Linearführung 41 in Form eines Langlochs versehen, das parallel zur Hebelebene 14 verläuft und in der Mittelebene 12 (Fig. 1) liegt. Die Mittelebene 12 verläuft durch die Drehachse 17 und mittig zwischen den beiden Tasthebeln 13, 15 hindurch. Längs der Langlochführung 41 sind die gelenkig miteinander verbundenen Enden zweier auch als Pleuel bezeichneter Gelenkhebel 38 zwangsgeführt, die mit ihren anderen Enden jeweils an die beiden Tasthebel 13, 15 angelenkt sind.

Durch diese erfindungsgemäße Geometrie wird erreicht, dass die beiden Tastbacken 23, 25 der Tasthebel 13, 15 unabhängig von der Schwenkstellung der beiden Tasthebel 13, 15 jeweils den gleichen Abstand von der Mittelebene 12 (Fig. 1) aufweisen. Dies bedeutet, dass die Symmetrieebene eines von den beiden Tastbacken 23, 25 gehaltenen Gegenstandes unabhängig davon, wie weit das Instrument geöffnet ist, stets mit der Mittelebene 12 des Instrumentes zusammenfällt.

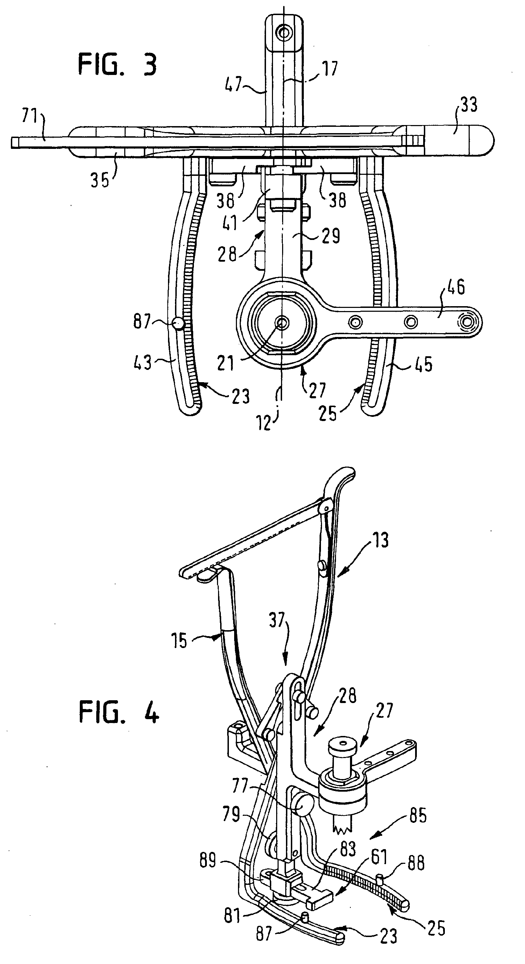

Der untere, auf der Tastseite der Drehachse 17 liegende Teil des Querbalkens des "T" ist im Wesentlichen von einer separaten Verlängerung 30 gebildet, die am Halter 28 durch eine Klemmschraube 77 gehalten ist. Ein Hilfsanschlag 61 ist an der Verlängerung 30 mittels einer weiteren Klemmschraube 79 (Fig. 4) gehalten und in der Mittelebene 12 parallel zur Hebelebene 14 verstellbar. Der Hilfsanschlag 61 weist ein separates Anschlagelement 83 auf, das wiederum in der Mittelebene 12, nunmehr jedoch senkrecht zur Hebelebene 14 und damit parallel zu einer von den Tastabschnitten 43, 45 der Tasthebel 13, 15 aufgespannten Tastebene 44 (Fig. 2) verstellbar. Das Anschlagelement 83 ist am Hilfsanschlag 61 mittels einer weiteren Klemmschraube 81 gehalten.

Zwei Peilstifte 87, 88 sind jeweils auf einer Tastbacke 23, 25 angeordnet. Ihre Verbindungslinie steht senkrecht zur Mittelebene 12 und kreuzt eine Bohrachse 21. Die Tastbacken 23, 25 werden beim Schließen des Instruments so verschoben, dass sie von der Seite gesehen etwa auf der Mitte des Femurhalses liegen. Das Anschlagelement 83 ist senkrecht zur Verbindungslinie der Peilstifte 87, 88 verschieblich einstellbar. Es besitzt eine Skala 89, welche jeweils den doppelten Abstand (zahlenmäßig) vom Hilfsanschlag 61 zur Verbindungslinie der Peilstifte 87, 88 anzeigt. Wenn in der Mittelebene 12 der Haldurchmesser in Zahlen - beispielsweise durch eine vorangehende Messung mit einer Schublehre - vorliegt, dann lässt sich dieser Durchmesser in Zahlen mit der Skala 89 einstellen und der Hilfsanschlag 61 sorgt dafür, dass zumindest im Bereich der Tastbacken 23, 25 die spätere Bohrachse 21 mit der Halsachse zusammenfällt.

Die vom Halter 28 am freien Ende des Trägers 29 getragene Funktionseinheit 27 umfasst eine weitere Peileinrichtung 46, 49 sowie eine Bohrlehre 31 mit Zentrierung 75.

Das freie Ende des Trägers 29 ist kreisringförmig ausgebildet. Auf dieses Ende ist mit einem ebenfalls kreisringförmigen Abschnitt ein vorderer Peilausleger 46 verschwenkbar aufgesteckt, dessen abstehender Peilarm mit einer Mehrzahl von Bohrungen versehen ist, die parallel zur Hebelebene 14 und damit senkrecht zur Tastebene 44 verlaufen. Die Bohrungen dienen zur Aufnahme eines weiteren Peilstabes 49.

Bei der Bohrlehre 31 handelt es sich um eine Hülse, die durch die Kreisringabschnitte des vorderen Peilauslegers 46 und des Trägerarmes 29 hindurchgeführt ist. Die untere Stirnseite der Hülse 31 ist mit einem gezahnten bzw. gezackten Rand versehen, der einen Zentrierabschnitt 75 bildet. Statt eines gezackten Randes können auch spitze, vorstehende Stifte vorgesehen sein. Des Weiteren weist die Hülse 31 eine zentrale Durchgangsbohrung auf, die eine Bohrachse 21 festlegt, welche in der Mittelebene 12 liegt und parallel zur Hebelebene 14 und somit senkrecht zur Tastebene 44 verläuft. Die Peilstäbe 49, 51 und ein durch die Bohrlehre 31 hindurch gestecktes, nicht dargestelltes Bohrinstrument, beispielsweise ein Kirschnerdraht, verlaufen somit parallel zueinander.

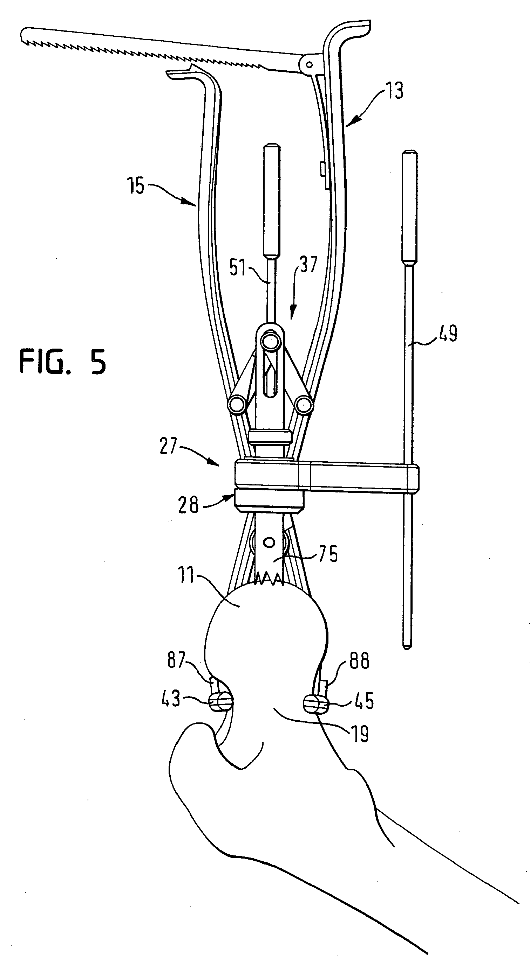

Im auf den Femur aufgesetzten Zustand (Fig. 5) liegen die Tastabschnitte 43, 45 am Femurhals 19 an. Der Femurkopf 11 befindet sich in einem Aufnahmeraum 85 des erfindungsgemäßen Instrumentes, der auf der einen Seite von der Tastebene 44 (Fig. 2), auf der gegenüberliegenden Seite von der Funktionseinheit 27 und senkrecht dazu von der Hebelebene 14 (Fig. 2) begrenzt ist. Damit wird von dem erfindungsgemäßen Instrument im Bereich des Femurkopfes 11 sehr wenig Platz benötigt.

In Fig. 5 nicht dargestellt ist der Hilfsanschlag 61 (Fig. 4), dessen Anschlagelement 83 in der Tastebene 44 (Fig. 2) liegt. Das freie Ende des Anschlagelementes 83 befindet sich somit ebenfalls in Höhe des Femurhalses 19 und stellt damit eine zusätzliche Ausrichthilfe bereit.

Das erfindungsgemäße Instrument dient zur Bestimmung von Ort und Richtung einer im Femurkopf 11 auszubildenden Bohrung für ein insbesondere draht- oder stiftförmiges Führungselement, entlang welchem anschließend Instrumente zur Bearbeitung des Femurkopfes 11 geführt werden können. Mit dem erfindungsgemäßen Instrument kann der Operateur über die an den Tastabschnitten 43, 45 ausgebildeten Tastbacken 23, 25 den Übergang zwischen Femurkopf 11 und Femurhals 19 ertasten und dabei in vorteilhafter und Platz sparender Weise von oben arbeiten.

Die Fixierung des erfindungsgemäßen Instrumentes am Femur ist ein iterativer Prozess, bei dem mit Hilfe der Peilstäbe 49, 51, von denen der vordere, auf der gleichen Seite der Hebelebene 14 wie die Funktionseinheit 27 liegende Peilstab 49 um die Bohrachse 21 verschwenkt werden kann, immer wieder die korrekte Ausrichtung der Bohrachse 21 kontrolliert werden kann. Von besonderem Vorteil ist, dass diese Überprüfung mit den beiden Peilstäben 49, 51 in zwei senkrecht zueinander stehenden Ebenen erfolgen kann. Mit dem Erreichen der Soll-Position kann das Instrument in dieser durch einen leichten Hammerschlag auf dem Zentrierabschnitt 75 fixiert werden.

Die Bedienung des erfindungsgemäßen Instrumentes ist für den Operateur extrem einfach und kann mit einer Hand erfolgen. Die während einer Schließbewegung erfolgende Verrastung der erreichten Schließstellung kann durch geringfügiges Anheben des Rastarmes 71 gelöst werden, um im Bedarfsfall das Instrument wieder öffnen und neu ansetzen zu können. Der Hilfsanschlag 61 sichert das Instrument gegen ein Verkippen in der Mittelebene 12. Eine grobe Voreinstellung des Hilfsanschlags 61 kann aufgrund der Operationsplanung erfolgen.

Die Zwangskoppelung der betätigungsseitigen Abschnitte der Tasthebel 13, 15 über die Gelenkhebel 38 mit der in der Mittelebene 12 verlaufenden Linearführung 41 sorgt automatisch für ein symmetrisches Öffnen und Schließen des Instrumentes bezüglich der Mittelebene 12. Damit ist sichergestellt, dass die Bohrachse 31 in dieser Mittelebene 12 liegt und damit zumindest näherungsweise durch den Mittelpunkt des Femurkopfes 11 verläuft, wenn die Tastbacken 23, 25 am Femurhals 19 bzw. am Halsansatz anliegen.

Ist die korrekte Ausrichtung des Instrumentes am Femurkopf 11 gefunden, kann durch Schläge auf die unten gezackte oder mit spitzen Stiften versehene Hülse der Bohrlehre 31 deren Zentrierung auf dem Femurkopf 11 erfolgen, wodurch das Instrument ausreichend sicher am Femurkopf 11 fixiert ist, um durch die zentrale Bohrung der Hülse 31 hindurch die gewünschte Bohrung in den Femurkopf 11 längs der ausgerichteten Bohrachse 21 einzubringen.

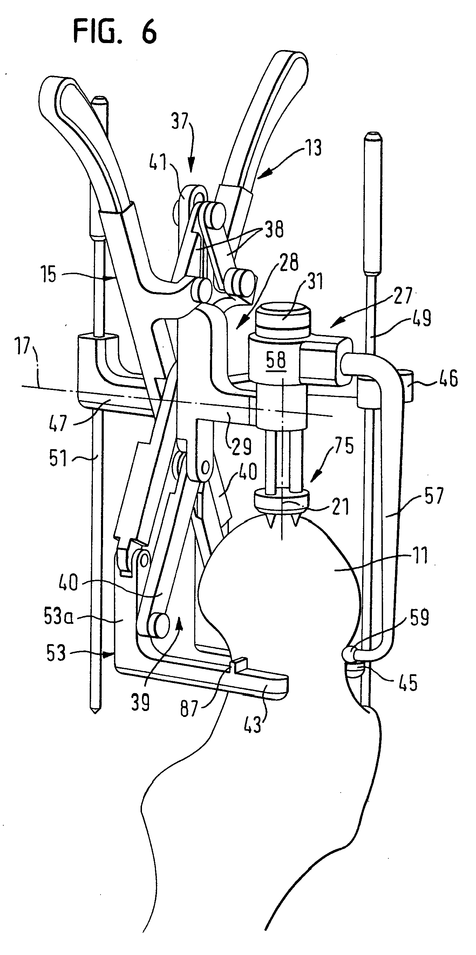

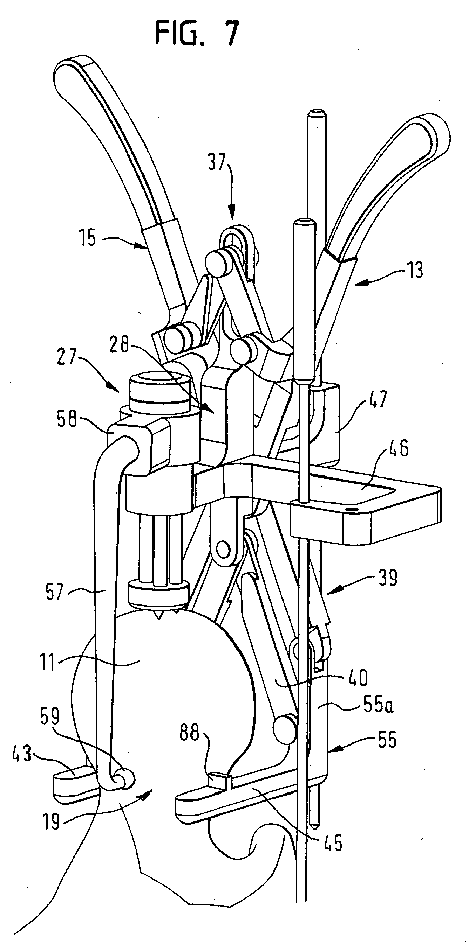

Das weitere Ausführungsbeispiel eines erfindungsgemäßen Instrumentes gemäß Fig. 6 und 7 unterscheidet sich von dem zuvor erläuterten Ausführungsbeispiel zunächst dadurch, dass die Tastabschnitte 43, 45 an verschwenkbaren Endabschnitten 53, 55 der Tasthebel 13, 15 vorgesehen sind, d.h. die Tasthebel 13, 15 sind nicht über ihre gesamte Länge starr ausgebildet, sondern mit einem Gelenk versehen. Die Schwenkachsen der Endabschnitte 53, 55 verlaufen senkrecht zur Hebelebene 14 (Fig. 2).

Die Schwenkbewegung der Endabschnitte 53, 55 ist durch eine weitere, unterhalb der Drehachse 17 gelegene Gelenkanordnung 39 zwangsgesteuert, die als Parallelogrammgelenk ausgebildet ist. Zwei Gelenkhebel 40, die jeweils mit ihrem einen Ende an einem parallel zur Hebelebene 14 verlaufenden Teilabschnitt 53a, 55a des verschwenkbaren Endabschnitts 53, 55 angelenkt sind, sind an ihren beiden anderen Enden gemeinsam an den Halter 28 angelenkt.

Eine in der Mittelebene 12 (Fig. 1) liegende Verbindungsgerade zwischen dem gemeinsamen Anlenkpunkt der beiden Gelenkhebel 40 und der Drehachse 17 verläuft parallel zu den Teilabschnitten 53a, 55a, wohingegen die Gelenkhebel 40 jeweils parallel zu demjenigen Teil des Tasthebels 13, 15 verlaufen, der sich zwischen der Drehachse 17 und dem Anlenkpunkt des verschwenkbaren Endabschnitts 53, 55 erstreckt. Hierdurch ist auf jeder Seite der Mittelebene 12 ein Viergelenkhebel in Form eines Parallelogramms gebildet. Beim Öffnen und Schließen des Instrumentes verlaufen die beiden Teilabschnitte 53a, 55a der verschwenkbaren Endabschnitte 53, 55 somit unabhängig von der Schwenkstellung der beiden Tasthebel 13, 15 stets parallel zueinander.

Des Weiteren ist in diesem Ausführungsbeispiel der vordere Peilausleger 46 für den vorderen Peilstab 49 nicht verschwenkbar, sondern bildet einen starren Arm des Halters 28. Der Peilausleger 46 ist U- bzw. hakenförmig ausgebildet, liegt in einer parallel zur Tastebene 44 (Fig. 2) verlaufenden Ebene liegt und ist um einen Schwenkbereich für einen nachstehend näher beschriebenen Tastarm 57 herumgeführt.

Der Tastarm 57 ist ein Bestandteil der Funktionseinheit 27 und mittels eines Schwenkteils 58 oberhalb der Drehachse 17 um die Bohrachse 21 herum verschwenkbar gelagert. Der Tastarm 57 erstreckt sich rüsselartig nach unten bis kurz oberhalb der von den Tastabschnitten 43, 45 aufgespannten Tastebene 44 und dabei um den Aufnahmebereich 85 (Fig. 4) des Instrumentes für den Femurkopf 11 herum.

Am freien Ende des Tastarmes 57 ist ein kugelförmiger Tastkopf 59 ausgebildet. Durch Verschwenken des Tastarmes 57 kann das so genannte "Auskreiseln" durchgeführt werden, bei dem der Tastkopf 59 um die Bohrachse 21 herum am Halsansatz des Femurkopfes 11 entlang geführt wird, um die Lage des Instrumentes in einer parallel zur Drehachse 17 verlaufenden Richtung relativ zum Femurkopf 11 zu kontrollieren. Hierzu wird das Instrument mit den Tastabschnitten 43, 45 etwas unterhalb des Halsansatzes am Femur fixiert, damit sich der Tastkopf 59 beim Auskreiseln in Höhe des Halsansatzes bewegen kann.

Ferner ist hier im Unterschied zu dem vorstehend erläuterten Ausführungsbeispiel der Zentrierabschnitt 75 nicht in Form einer "kronenartigen" Stirnseite einer Hülse vorgesehen. Vielmehr wird hier der Zentrierabschnitt 75 von drei parallelen, unten spitz zulaufenden Stiften gebildet, die im gleichen radialen Abstand von der Bohrachse 21 und gleichmäßig in Umfangsrichtung voneinander beabstandet angeordnet sind.

Eine Auskreiseleinheit entsprechend dem Ausführungsbeispiel der Fig. 6 und 7 kann auch bei dem Ausführungsbeispiel gemäß Fig. 1 bis 5 vorgesehen sein. Des Weiteren kann bei beiden Ausführungsbeispielen auch jeweils die andere Bohrlehre 31 bzw. der jeweils andere Zentrierabschnitt 75 vorgesehen sein. Ein Parallelogrammgelenk mit verschwenkbaren Endabschnitten entsprechend dem Ausführungsbeispiel der Fig. 6 und 7 kann auch bei dem Ausführungsbeispiel der Fig. 1 bis 5 vorgesehen sein.

### Bezugszeichenliste

- 11: Femurkopf
- 12: Mittelebene
- 13: Tasthebel, Klemme
- 14: Hebelebene
- 15: Tasthebel, Klemme
- 17: Gelenkbereich, Drehachse
- 19: Femurhals
- 21: Peil- und/oder Bohrachse
- 23: Tastbacke
- 25: Tastbacke
- 27: Funktionseinheit
- 28: Halter
- 29: Träger
- 30: Verlängerung
- 31: Bohrlehre
- 33: Betätigungsabschnitt
- 35: Betätigungsabschnitt
- 37: Gelenkhebelanordnung
- 38: Gelenkhebel
- 39: Gelenkhebelanordnung, Parallelogrammgelenk
- 40: Gelenkhebel
- 41: Linearführung
- 43: Tastabschnitt
- 44: Tastebene
- 45: Tastabschnitt
- 46: vorderer Peilausleger
- 47: hinterer Peilausleger
- 49: vorderer Peilstab

- 51: hinterer Peilstab
- 53: verschwenkbarer Endabschnitt
- 53a: Teilabschnitt
- 55: verschwenkbarer Endabschnitt
- 55a: Teilabschnitt
- 57: Tastarm
- 58: Schwenkteil
- 59: Tastkopf
- 61: Hilfsanschlag
- 71: Rastarm
- 73: Feder
- 75: Zentrierabschnitt
- 77: Klemmschraube
- 79: Klemmschraube
- 81: Klemmschraube
- 83: Anschlagelement
- 85: Aufnahmeraum
- 87: Peilstift
- 88: Peilstift
- 89: Skala

## Patentansprüche

1. Instrument zum Vorbereiten und/oder Bearbeiten eines Femurkopfes (11), insbesondere zum Festlegen einer bezüglich des Femurhalses zentralen Bohrung für ein bevorzugt draht- oder stabförmiges Führungselement eines Bearbeitungswerkzeuges,
mit zwei nach Art einer Schere oder Zange gelenkig miteinander verbundenen Tasthebeln (13, 15), die jeweils auf der einen Seite des Gelenkbereiches (17) eine Tastbacke (23, 25) zum Ertasten des Femurhalses (19) und auf der anderen Seite des Gelenkbereiches (17) einen Betätigungsabschnitt (33, 35) zum Öffnen und Schließen der Tastbacken (23, 25) aufweisen, und
mit einer eine Peil- und/oder Bohrachse (21) definierenden Funktionseinheit (27), die mit den Tasthebeln (13, 15) derart verbunden ist, dass die Peil- und/oder Bohrachse (21) unabhängig von der Stellung der Tasthebel (13, 15) stets in einer durch den Gelenkbereich (17) und mittig zwischen den Tastbacken (23, 25) hindurch verlaufenden Mittelebene (12) liegt.

2. Instrument nach Anspruch 1,
**dadurch gekennzeichnet,**
**dass** der Gelenkbereich eine in der Mittelebene (12) liegende Drehachse (17) umfasst, um welche die beiden Tasthebel (13, 15) zum Öffnen und Schließen der Tastbacken (23, 25) gegeneinander verdrehbar sind.

3. Instrument nach Anspruch 1 oder 2,
**dadurch gekennzeichnet,**
**dass** die beiden Tasthebel (13, 15) zumindest in einer wenigstens teilweise geöffneten Stellung eine X-förmige Grundstruktur bilden.

4. Instrument nach einem der vorhergehenden Ansprüche,
**dadurch gekennzeichnet,**
**dass** die Mittelebene (12) und eine von den beiden Tasthebeln (13, 15) aufgespannte Hebelebene (14) senkrecht aufeinander stehen.

5. Instrument nach einem der vorhergehenden Ansprüche,
**dadurch gekennzeichnet,**
**dass** die Peil- und/oder Bohrachse (21) parallel zu einer von den beiden Tasthebeln (13, 15) aufgespannten Hebelebene (14) verläuft.

6. Instrument nach einem der vorhergehenden Ansprüche,
**dadurch gekennzeichnet,**
**dass** die Funktionseinheit (27) einer von den beiden Tasthebeln (13, 15) aufgespannten Hebelebene (14) vorgelagert ist.

7. Instrument nach einem der vorhergehenden Ansprüche,
**dadurch gekennzeichnet,**
**dass** die Tastbacken (23, 25) einer von den beiden Tasthebeln (13, 15) aufgespannten Hebelebene (14) vorgelagert sind, wobei vorzugsweise die Tastbacken (23, 25) und die Funktionseinheit (27) sich auf der gleichen Seite der Hebelebene (14) befinden.

8. Instrument nach einem der vorhergehenden Ansprüche,
**dadurch gekennzeichnet,**
**dass** die Tastbacken (23, 25) an vorzugsweise etwa senkrecht von den Tasthebeln (13, 15) abstehenden Tastabschnitten (43, 45) ausgebildet sind.

9. Instrument nach einem der vorhergehenden Ansprüche,
**dadurch gekennzeichnet,**
**dass** die Funktionseinheit (27) über einen in der Mittelebene (12) liegenden Träger (29) mit den Tasthebeln (13, 15) verbunden ist, wobei vorzugsweise sich der Träger (29) senkrecht zu einer von den beiden Tasthebeln (13, 15) aufgespannten Hebelebene erstreckt.

10. Instrument nach Anspruch 9,
**dadurch gekennzeichnet,**
**dass** der Träger (29) mit einer Drehachse (17) zusammenfällt, um welche die beiden Tasthebel (13, 15) zum Öffnen und Schließen der Tastbacken (23, 25) gegeneinander verdrehbar sind.

11. Instrument nach einem der vorhergehenden Ansprüche,
**dadurch gekennzeichnet,**
**dass** die Tasthebel (13, 15) um eine feste Drehachse (17) gegeneinander verdrehbar und mit Abstand von der Drehachse (17) zusätzlich über wenigstens eine Gelenkhebelanordnung (37, 39) miteinander verbunden sind.

12. Instrument nach einem der vorhergehenden Ansprüche,
**dadurch gekennzeichnet,**
**dass** die Gelenkhebelanordnung (37) eine in der Mittelebene (12) liegende Linearführung (41) für sich beim Öffnen und Schließen der Tastbacken (23, 25) bewegende Gelenkhebel (38) umfasst.

13. Instrument nach Anspruch 12,
**dadurch gekennzeichnet,**
**dass** die Linearführung (41) und die Tastbacken (23, 25) sich auf unterschiedlichen Seiten des Gelenkbereiches (17) befinden.

14. Instrument nach Anspruch 12 oder 13,
**dadurch gekennzeichnet,**
**dass** ein Halter (28) vorgesehen ist, der die Funktionseinheit (27) trägt und mit den Tasthebeln (13, 15) verbindet und an dem die Linearführung (41) insbesondere in Form eines Langlochs ausgebildet ist.

15. Instrument nach einem der vorhergehenden Ansprüche,
**dadurch gekennzeichnet,**
**dass** die Tastbacken (23, 25) an verschwenkbaren Endabschnitten (53, 55) der Tasthebel (13, 15) ausgebildet sind.

16. Instrument nach Anspruch 15,
**dadurch gekennzeichnet,**
**dass** die verschwenkbaren Endabschnitte (53, 55) der Tasthebel (13, 15) durch eine Gelenkhebelanordnung (39) miteinander verbunden sind.

17. Instrument nach Anspruch 16,
**dadurch gekennzeichnet,**
**dass** die Gelenkhebelanordnung (39) derart ausgebildet ist, dass Teilabschnitte (53a, 55a) der verschwenkbaren Endabschnitte (53, 55), die sich parallel zu einer von den beiden Tasthebeln (13, 15) aufgespannten Hebelebene (14) erstrecken, in jeder Stellung die gleiche Orientierung relativ zueinander aufweisen und insbesondere parallel zueinander verlaufen, wobei vorzugsweise die Gelenkhebelanordnung (39) nach dem Parallelogrammprinzip ausgeführt ist.

18. Instrument nach einem der vorhergehenden Ansprüche,
**dadurch gekennzeichnet,**
**dass** die Funktionseinheit (27) eine Bohrlehre (31) zum Festlegen einer mit der Peil- und/oder Bohrachse (21) zusammenfallenden Bohrung im Femurkopf (11) umfasst.

19. Instrument nach einem der vorhergehenden Ansprüche,
**dadurch gekennzeichnet,**
**dass** die Funktionseinheit (27) wenigstens einen Peilausleger (46, 47) aufweist, mit dem zumindest ein Peilstab (49, 51) derart koppelbar ist, dass der Peilstab (49, 51) sich parallel zur Peil- und/oder Bohrachse (21) erstreckt.

20. Instrument nach Anspruch 19,
**dadurch gekennzeichnet,**
**dass** ein bevorzugt relativ zur Funktionseinheit (27) stationärer Peilausleger (47) und die Peil- und/oder Bohrachse (21) sich auf unterschiedlichen Seiten einer von den beiden Tasthebeln (13, 15) aufgespannten Hebelebene (14) befinden.

21. Instrument nach Anspruch 19 oder 20,
**dadurch gekennzeichnet,**
**dass** ein Peilausleger (46) um eine parallel versetzt zur Peil- und/oder Bohrachse (21) verlaufende oder mit der Peil- und/oder Bohrachse (21) zusammenfallende Achse relativ zur Funktionseinheit (27) verschwenkbar ist.

22. Instrument nach einem der vorhergehenden Ansprüche,
**dadurch gekennzeichnet,**
**dass** die Funktionseinheit (27) einen um die Peil- und/oder Bohrachse (21) verschwenkbaren Tastarm (57) zum Abtasten des Femurhalses (19) und/oder des Übergangs zwischen Femurkopf (11) und Femurhals (19) umfasst.

23. Instrument nach Anspruch 21,
**dadurch gekennzeichnet,**
**dass** der Tastarm (57) sich um einen oberhalb der Tastbacken (23, 25) gelegenen und einer von den beiden Tasthebeln (13, 15) aufgespannten Hebelebene (14) vorgelagerten Aufnahmebereich (85) für den Femurkopf (11) herum erstreckt und insbesondere U-förmig mit der Hebelebene (14) zugewandter offener Seite ausgebildet ist.

24. Instrument nach Anspruch 22 oder 23,
**dadurch gekennzeichnet,**
**dass** ein insbesondere das freie Ende des Tastarmes (57) bildender Tastkopf (59) des Tastarmes (57) etwa in Höhe der Tastbacken (23, 25) gelegen ist.

25. Instrument nach einem der vorhergehenden Ansprüche,
**dadurch gekennzeichnet,**
**dass** ein Peilausleger (46, 47) und/oder ein Tastarm (57) lösbar mit der Funktionseinheit (27) koppelbar sind.

26. Instrument nach einem der vorhergehenden Ansprüche,
**dadurch gekennzeichnet,**
**dass** ein in der Mittelebene (12) vorzugsweise in zwei senkrecht zueinander verlaufenden Richtungen relativ zur Funktionseinheit (27) verstellbarer Hilfsanschlag (61) vorgesehen ist.

27. Instrument nach einem der vorhergehenden Ansprüche,
**dadurch gekennzeichnet,**
**dass** ein Aufnahmeraum (85) für den Femurkopf (11) von einer Tastebene (44), in der die Tastbacken (23,25) liegen, auf der gegenüberliegenden Seite von der Funktionseinheit (27) und im Wesentlichen senkrecht dazu von einer Hebelebene (14), die von den Tasthebeln (13, 15) aufgespannt ist, begrenzt ist.

28. Instrument nach einem der vorhergehenden Ansprüche,
**dadurch gekennzeichnet,**
**dass** an den Tastbacken 23, 25 Peilstifte 87, 88 angebracht sind, die senkrecht mit ihrer Verbindungslinie zur Mittelebene 12 stehen und die Bohrachse 21 kreuzen.
